# EUROPÄISCHE PATENTSCHRIFT

(11) **EP 0 767 163 B1**
(45) Veröffentlichungstag und Bekanntmachung des Hinweises auf die Patenterteilung: **10.01.2001**
(21) Anmeldenummer: 96115453.1
(22) Anmeldetag: 26.09.1996
(51) Int. Cl.: C07C 67/327, C07C 69/54, C07C 67/08, C07C 67/60

(54) **Verfahren zum Verestern von (Meth)acrylsäure mit einem Alkanol**
Process for esterifying (meth)acrylic acid with an alcanol
Procédé d'estérification de l'acide (méth)acrylique avec un alcanol

(30) Priorität: 28.09.1995 DE 19536191
(43) Veröffentlichungstag der Anmeldung: 09.04.1997
(73) Patentinhaber: BASF AKTIENGESELLSCHAFT, 67056 Ludwigshafen (DE)
(72) Erfinder: Aichinger, Heinrich Dr., 68199 Mannheim (DE); Geisendörfer, Matthias, Dr., 67435 Neustadt (DE); Herbst, Holger, Dr., 67227 Frankenthal (DE); Nestler, Gerhard, Dr., 67061 Ludwigshafen (DE)
(74) Vertreter: Isenbruck, Günter, Dr.

(56) Entgegenhaltungen:
- DE-A- 2 339 519
- DE-A- 19 547 485

## Beschreibung

Die Erfindung betrifft ein Verfahren zum Verestern von (Meth)acrylsäure mit einem Alkanol in Gegenwart eines Veresterungskatalysators, bei dem nicht umgesetzte Ausgangsverbindungen und der zu bildende (Meth)acrylsäure-ester destillativ abgetrennt werden und ein Oxyester enthaltendes Sumpfprodukt entsteht. Der Begriff (Meth)acrylsäure bezeichnet dabei in bekannter Weise Acryl- oder Methacrylsäure.

Die Herstellung von Alkylestern der (Meth)acrylsäure erfolgt üblicherweise durch Verestern von (Meth)acrylsäure mit Alkanolen bei erhöhter Temperatur in flüssiger Phase mit oder ohne Lösungsmittel und in Gegenwart von Säure als Katalysator (DE-A 23 39 519). Nachteilig an dieser Herstellungsweise ist, daß sich unter den vorgenannten Veresterungsbedingungen als Nebenreaktionen noch nicht umgesetzter Ausgangsalkohol unter Ausbildung einer Verbindung der nachfolgend angeführten allgemeinen Formel I sowie noch nicht umgesetzte (Meth)acrylsäure unter Ausbildung einer Verbindung der allgemeinen Formel II an die Doppelbindung von bereits gebildetem (Meth)acrylsäurealkylester addiert (Michael-Addition). Auch eine Mehrfachaddition ist möglich. Ferner können gemischte Typen auftreten. Diese Addukte (Alkoxyester und Acyloxyester) nennt man kurz Oxyester. 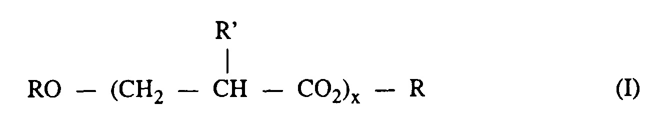 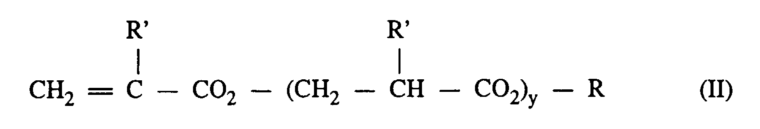 mit
- x,y =: 1-5
- R =: Alkyl
- R' =: H oder CH₃

Wenn R' = H ist, handelt es sich um eine Veresterung der Acrylsäure, wenn R' = CH₃ ist, handelt es sich um eine Veresterung der Methacrylsäure.

Bei der Herstellung von Estern der Acrylsäure ist das Problem der Oxyesterbildung besonders akut, wobei die hauptsächlich gebildeten Oxyester der Alkoxypropionsäureester und der Acyloxypropionsäureester mit x,y = 1 sind. Bei der Herstellung von Estern der Methacrylsäure erfolgt die Oxyesterbildung in geringerem Maße. Die Entstehung von Oxyestern ist in der DE-A 23 39 529 beschrieben. Aus dieser geht hervor, daß die Bildung von Oxyestern im wesentlichen unabhängig von den speziellen Veresterungsbedingungen erfolgt. Von ganz besonderer Bedeutung ist die Oxyesterbildung bei der Herstellung von Acrylaten der C₁- bis C₈-Alkanole, insbesondere der C₄- bis C₈-Alkanole, ganz besonders bei der Herstellung von n-Butylacrylat und 2-Ethylhexylacrylat.

Charakteristisch für die Oxyester ist, daß ihr Siedepunkt oberhalb der Siedepunkte von Ausgangssäure, Ausgangsalkohol, gebildetem Zielester sowie gegebenenfalls mitverwendetem organischem Lösungsmittel liegt.

Die Aufarbeitung eines beliebigen Veresterungs-Reaktionsgemisches erfolgt normalerweise so, daß nicht umgesetzte Ausgangsverbindungen sowie der Zielester vom Reaktionsgemisch destillativ abgetrennt werden, wobei der zur Veresterung verwendete Säurekatalysator gegebenenfalls vorher durch Extraktion mittels Wasser und/oder wäßriger Lauge abgetrennt wird (vgl. z.B. Ullmann's Encyclopedia of Industrial Chemistry, Vol. A1, 5th Ed., VCH, S. 167ff). Das im Rahmen einer solchen destillativen Aufarbeitung verbleibende Sumpfprodukt enthält die Oxyester, die einen beträchtlichen Ausbeuteverlust bedingen.

Es sind daher weitere verschiedenartige Verfahren untersucht worden, um die durch das Auftreten der Oxyester entstehenden Probleme zu lösen. So beschreibt die JP-A-82/62229 die alkalische Verseifung des hochsiedenden Veresterungsrückstandes. Man erhält auf diese Weise einen Teil des eingesetzten Alkohols und Acrylsäure und β-Hydroxypropionsäure bzw. deren Salze zurück. Eine einfache und wirtschaftliche Rückführung der Produkte in die Veresterungsreaktion ist daher nicht möglich. Die JP-AS-72/15936 beschreibt die Gewinnung von Acrylestern durch Umsetzung von β-Alkoxypropionsäureestern mit Acrylsäure in Gegenwart von starken Säuren (Umesterung). Als Nebenprodukt fallen dabei jedoch äquimolare Mengen an β-Alkoxypropionsäure an, die nicht in die Veresterungsreaktion zurückgeführt werden können und daher Abfall darstellen. Die JP-A-93/25086 beschreibt die Spaltung des Michael-Additionsproduktes β-Butoxypropionsäurebutylesters (s. Formel I, x = 1, R = Butyl) bei erhöhter Temperatur und in Gegenwart von Schwefelsäure und eines Überschusses an Wasser. Der Umsatz beträgt jedoch lediglich ca. 30 %. Schließlich beschreibt JP-A-94/65149 die Spaltung der Michael-Additionsprodukte I und II (s.o. x = y = 1) in Gegenwart von Titanalkoholaten. Hier ist der Umsatz ebenfalls gering (< 60%) und es sind hohe Titanatmengen notwendig. Daher ist dieses Verfahren unwirtschaftlich und wegen der großen zu entsorgenden Titanatmengen umweltbelastend.

In der GB 923 595 ist die Gewinnung von Monomeren aus dem Rückstand der Veresterung von Acrylsäure mit Alkanolen in Abwesenheit von molekularem Sauerstoff beschrieben. Empfohlen wird u.a. die Entfernung von allen flüchtigen Monomeren vor der Spaltung, die Spaltung in Gegenwart von Schwefelsäure und die Entfernung der Spaltprodukte mit Hilfe eines Inertgasstromes. Entsprechend den Ausführungsbeispielen wird die Spaltung immer bei min. 300 °C durchgeführt. Als Rückstand entsteht Koks (17-40%). Dieser muß "bergmännisch" aus dem Reaktor entfernt werden. Daher ist dieses Verfahren weder wirtschaftlich noch im technischen Maßstab durchführbar. Ein weiterer Nachteil ist der notwendige Sauerstoffausschluß.

Die CN-A 1,063,678 beschreibt die Spaltung des im Veresterungsrückstand enthaltenen Alkoxypropionsäureesters in Gegenwart von Schwefelsäure in einer Kaskade, wobei Temperatur und Katalysatorkonzentration (0,8 bis 1,5 %) in jedem Reaktor unterschiedlich sind. Gekoppelt mit der Spaltung ist eine Destillation zur Trennung von Alkanol und Acrylat. Das Verfahren ist sehr umständlich und erreicht keine hohen Umsätze.

Schließlich beschreibt die CN-A 105 8390 die Spaltung von Alkoxypropionsäureestern in Gegenwart von Schwefelsäure etc. in Alkanole und Acrylsäureester. Es wird dabei schrittweise vorgegangen. Zuerst wird die Spaltung unter Rückfluß durchgeführt und anschließend die Reaktionsprodukte abdestilliert. Die Spaltung der acrylsäurehaltigen Esterrückstände der Ethyl/Methylacrylatherstellung (Ethyl-ethoxypropionat, Methyl-methoxypropionat) wird in Gegenwart von Ethanol bzw. Methanol durchgeführt. Auch hier ist das Verfahren kompliziert und erreicht keine hohen Umsätze.

Der Erfindung liegt die Aufgabe zugrunde, die Rückspaltung der in diesem Sumpfprodukt enthaltenen Oxyester und die Weiterverwendung der dabei anfallenden Ausgangssäure, Ausgangsalkohol und Zielester im Rahmen der Veresterung ohne die Nachteile der Verfahren nach dem Stand der Technik durchzuführen.

Diese Aufgabe wird erfindungsgemäß dadurch gelöst, daß das Sumpfprodukt abgetrennt und die in ihm enthaltenen Oxyester in Anwesenheit von Säure auf 150 bis 250 °C erhitzt wird und der Druck so eingestellt wird, daß die unter den vorgenannten Bedingungen aus den im Sumpfprodukt enthaltenen Oxyestern entstehenden Spaltprodukte abdampfen. Dabei wird das Verfahren im Beisein von molekularem Sauerstoff durchgeführt, indem als Schleppmittel für die Spaltprodukte ein Strippgas, das molekularen Sauerstoff enthält, durch das Sumpfprodukt geführt wird.

Gemäß einer vorteilhaften Weiterbildung der Erfindung werden dem Sumpfprodukt zusätzlich zu dem ggf. bereits enthaltenen sauren Veresterungskatalysator weitere Säuren, beispielsweise Mineralsäuren, wie Schwefelsäure oder Phosphorsäure, und/oder organische Säuren wie Alkyl- oder Arylsulfonsäuren, beispielsweise Methansulfonsäure oder p-Toluolsulfonsäure, zugesetzt. Dabei kann die dann insgesamt enthaltene Säuremenge 1 bis 20 Gew.-%, vorzugsweise 5 bis 15 Gew.-%, bezogen auf die Mengen des Sumpfprodukts, betragen. Zweckmäßigerweise werden als Strippgas Luft oder Gemische von Luft mit Inertgas (z.B. Stickstoff) verwendet.

Für die Aufarbeitung der bei der Veresterung als Sumpfprodukt anfallenden Oxyester kann ein einfacher beheizbarer Rührreaktor mit Doppelwandheizung oder Heizschlange, oder auch ein Zwangsumlaufverdampfer, beispielsweise ein Fallfilmverdampfer oder Flashverdampfer, gekoppelt mit einem Verweilzeitbehälter verwendet werden. Zur besseren Trennung der Spaltprodukte vom Sumpfprodukt ist ggf. eine auf die Spaltapparatur aufgesetzte Rektifikationsvorrichtung, z.B. eine Füllkörper-, Packungs- oder Bodenkolonne zweckmäßig. Diese Rektifikationsvorrichtung wird in der Regel mit Polymerisationsinhibitoren (z.B. Phenothiazin, Hydrochinonmonomethylether etc.) stabilisiert betrieben.

Die Bedingungen für die Durchführung des erfindungsgemäßen Verfahrens der Spaltung der bei der Veresterung als Sumpfprodukt angefallenen Oxyestern sind folgende:
- Katalysator:: wenigstens eine Säure aus der Gruppe umfassend Mineralsäuren, wie z.B. Schwefelsäure und Phosphorsäure, sowie organische Säuren wie z.B. Alkyl- oder Arylsulfonsäuren, beispielsweise Methansulfonsäure oder p-Toluolsulfonsäure
- Katalysatormenge:: 1 - 20 Gew.-%, vorzugsweise 5 - 15 Gew.-% bezogen auf die Menge des Sumpfprodukts
- Temperatur:: 150 - 250 °C, vorzugsweise 180 - 230 °C
- Druck:: vorzugsweise drucklos bzw. reduzierter Druck
- Strippgas: Menge: 1 - 100 l/h 1
- Reaktionszeit:: 1 - 10 Std.
- Umsatz:: in der Regel ca. 80%

Die Reaktionsführung läuft z.B. in der Weise, daß das zu spaltende Sumpfprodukt kontinuierlich aus der destillativen Aufarbeitung des Veresterungsgemisches ausgeschleust und mit dem Spaltkatalysator dem Spaltreaktor zugeführt wird. Die Reaktionsführung kann aber auch diskontinuierlich, d.h. batchweise, durchgeführt werden. Auch eine halbkontinuierliche Reaktionsführung ist möglich, bei der das zu spaltende Produkt kontinuierlich dem Spaltreaktor, der den Spaltkatalysator enthält, zugeführt wird und das Sumpfprodukt erst nach Beendigung der Spaltung aus dem Spaltreaktor batchweise entfernt wird. Die Spaltprodukte werden kontinuierlich destillativ abgetrennt.

Die Anwendbarkeit des beschriebenen Spaltverfahrens ist nicht auf eine spezielle Natur des Veresterungsprozesses, als dessen Nebenprodukte die Oxyester, also die Additionsverbindungen I und II, anfallen, beschränkt. In der Regel werden die Ester nach den üblichen Verfahren hergestellt (s. Ullmann's Encyclopedia of Industrial Chemistry, Volume A1, 5th Ed., VCH, S. 167ff).

Ein typisches Beispiel für die Bedingungen, unter denen die der Spaltung der Oxyester vorausgehende Veresterung stattfinden kann, lassen sich kurz wie folgt darstellen:
- Alkohol:: (Meth)acrylsäure 1 : 0,7 - 1,2 (molar)
- Katalysator:: Schwefelsäure oder Sulfonsäuren
- Katalysatormenge:: 0,1 - 10 Gew.-% (vorzugsweise 0,5 - 5 Gew. %) bzgl. Einsatzstoffe
- Stabilisierung:: 200 - 2000 ppm Phenothiazin (bezogen auf das Gewicht der Einsatzstoffe)
- Reaktionstemperatur:: 80 - 160 °C, vorzugsweise 90 - 130 °C
- Reaktionszeit:: 1 - 10 Std., vorzugsweise 1 - 6 Std.

Gegebenenfalls wird ein Schleppmittel (z.B. Cyclohexan oder Toluol) zur Entfernung des Veresterungswassers eingesetzt. Die Veresterung kann drucklos, mit Überdruck oder Unterdruck sowohl kontinuierlich als auch diskontinuierlich durchgeführt werden.

Bei der säurekatalysierten Veresterung von Acrylsäure mit Alkanolen hat das nach der Abtrennung des sauren Veresterungskatalysators, der nicht umgesetzten Ausgangsstoffe und des Acrylesters resultierende Sumpfprodukt in der Regel folgende Zusammensetzung:
1 - 20 Gew.-% Acrylester
50 - 80 Gew.-% Alkoxypropionate (s. Formel I)
5 - 30 Gew.-% Acyloxypropionate (s. Formel II)
Rest: hauptsächlich Stabilisatoren (Phenothiazin) und Polymere

Weitere Einzelheiten und Vorteile des erfindungsgemäßen Verfahrens können den im folgenden beschriebenen Ausführungsbeispielen entnommen werden.

### Beispiel 1 (Vergleich)

Ein aus Glas bestehender Umlaufreaktor (Volumen: 1 l), beheizt mit einer Heizkerze, wurde mit 40 g p-Toluolsulfonsäure und 500 g eines vom sauren Veresterungskatalysator befreiten Veresterungsrückstandes der n-Butylacrylatherstellung gefüllt. Der Rückstand enthielt 10,1 Gew.-% Butylacrylat, 65,4 Gew.-% Butoxyester I und 20,0 Gew.-% Acyloxyester II (R = C₄H₉). Der Rest bestand aus Polymeren, Oligomeren und Polymerisationsinhibitor (Phenothiazin). Die Spalttemperatur betrug 195 °C und der Arbeitsdruck lag bei 1 atm.

Standgeregelt wurde dem Spaltreaktor während der Spaltung kontinuierlich Veresterungsrückstand zugeführt.

Die Spaltprodukte wurden dampfförmig abgeführt und kondensiert. Zwischen Reaktor und Kondensator befand sich eine leere Kolonne (50 cm x 2,8 cm) als Spritzschutz. Innerhalb von 21,5 Stunden wurden auf diese Weise 1589 g Veresterungsrückstand der Spaltung zugeführt. Entsprechend der Gaschromatographischen Analyse enthielt das anfallende Kondensat (1278 g):
69,1 Gew.-% Butylacrylat
18,3 Gew.-% Butanol
6,5 Gew.-% Acrylsäure
7,0 Gew.-% Olefine und Ether
3,5 Gew.-% Butoxypropionsäurebutylester
Umsatz: 84 Gew.-% bezogen auf Oxyester

### Beispiel 2

Eine Spaltapparatur, bestehend aus einem 1 l Rührreaktor, einer aufgesetzten Kolonne (30 cm x 2,8 cm, 5 mm Raschigringe) und einem Kondensator wurde mit 15 g p-Toluolsulfonsäure und 500 g einer bei der Herstellung von 2-Ethylhexylacrylat anfallenden, keinen sauren Veresterungskatalysator mehr aufweisenden, Sumpfflüssigkeit folgender Zusammensetzung gefüllt:
65,0 Gew.-% Alkoxyester I (R = C₈H₁₇)
5,5 Gew.-% Acyloxyester II (R = C₈H₁₇)
2,1 Gew.-% 2-Ethylhexylacrylat
1,0 Gew.-% Di-2-ethylhexylether
Rest: Polymere, Oligomere, Polymerisationsinhibitor (Phenothiazin)

Die Spalttemperatur betrug 215 °C und der Arbeitsdruck lag bei 1 atm.. Während der Spaltung werden 100 l Luft/h als Strippgas kontinuierlich durchgeleitet. Reaktionszeit 1,5 Stunden. Das Kondensat (288 g) enthielt entsprechend der gaschromatographischen Analyse:
7,2 Gew.-% Acrylsäure
25,5 Gew.-% 2-Ethylhexanol
53,0 Gew.-% 2-Ethylhexylacrylat
1,9 Gew.-% Di-2-ethylhexylether
12,2 Gew.-% Octene
< 1 Gew.-% Alkoxyester I (R = C₈H₁₇)
Umsatz: 79 Gew.-% bezogen auf Oxyester

### Beispiel 3 (Vergleich)

Eine Spaltapparatur, bestehend aus einem 1 l Rührreaktor, einer aufgesetzten Kolonne (30 cm x 2,8 cm, 5 mm Raschigringe) und einem Kondensator wurde mit 25 g p-Toluolsulfonsäure und 500 g einer bei der Herstellung von 2-Ethylhexylacrylat anfallenden, keinen sauren Veresterungskatalysator mehr aufweisenden, Sumpfflüssigkeit folgender Zusammensetzung gefüllt:
65,0 Gew.-% Alkoxyester I (R = C₈H₁₇)
5,5 Gew.-% Acyloxyester II (R = C₈H₁₇)
2,1 Gew.-% 2-Ethylhexylacrylat
1,0 Gew.-% Di-2-ethylhexylether
Rest: Polymere, Oligomere, Veresterungsstabilisator (Phenothiazin)

Die Spalttemperatur betrug 190 °C bei einem Druck von 50 mbar. Das Kondensat (321 g) enthielt entsprechend der gaschromatographischen Analyse:
4,2 Gew.-% Acrylsäure
27,9 Gew.-% 2-Ethylhexanol
52,2 Gew.-% 2-Ethylhexylacrylat
2,7 Gew.-% Di-2-ethylhexylether
15,5 Gew.-% Octene
< 1 Gew.-% Alkoxyester I (R = C₈H₁₇)
Umsatz: 88 Gew.-% bezogen auf Oxyester

Den Versuchsbeispielen kann entnommen werden, daß mit dem erfindungsgemäßen Verfahren Rückspaltumsätze von ca. 80 Gew.-% und mehr erzielt werden können.

## Patentansprüche

1. Verfahren zum Verestern von (Meth)acrylsäure mit einem Alkanol in Gegenwart eines sauren Veresterungskatalysators, bei dem nicht umgesetzte Ausgangsverbindungen und der zu bildende (Meth)acrylsäureester destillativ abgetrennt werden und ein Oxyester enthaltendes Sumpfprodukt entsteht, **dadurch gekennzeichnet, daß** das Sumpfprodukt abgetrennt und in Anwesenheit von Säure auf 150 bis 250 °C erhitzt und der Druck so eingestellt wird, daß die unter den vorgenannten Bedingungen aus den im Sumpfprodukt enthaltenen Oxyestern entstehenden Spaltprodukte unmittelbar abdampfen, wobei das Verfahren im Beisein von molekularem Sauerstoff durchgeführt wird, indem als Schleppmittel für die Spaltprodukte ein Strippgas, das molekularen Sauerstoff enthält, durch das Sumpfprodukt geführt wird.

2. Verfahren nach Anspruch 1, dadurch gekennzeichnet, daß dem Sumpfprodukt Mineralsäuren, beispielsweise Schwefelsäure oder Phosphorsäure, und/oder Alkyl- oder Arylsulfonsäuren, beispielsweise Methansulfonsäure oder p-Toluolsulfonsäure, zugesetzt werden.

3. Verfahren nach Anspruch 1 oder 2, dadurch gekennzeichnet, daß die enthaltene Säuremenge bezogen auf die Menge des Sumpfprodukts 1 bis 20 Gew.-%, vorzugsweise 5 bis 15 Gew.-%, beträgt.

4. Verfahren nach einem der Ansprüche 1 bis 3, dadurch gekennzeichnet, daß bei der Spaltung reduzierter Druck (< 1 atm) angewendet wird.

5. Verfahren nach einem der Ansprüche 1 bis 4, dadurch gekennzeichnet, daß die anfallenden Spaltprodukte unmittelbar in die Veresterung rückgeführt werden.

6. Verfahren nach einem der Ansprüche 1 bis 5, dadurch gekennzeichnet, daß es sich bei dem Sumpfprodukt um das bei der Veresterung von n-Butanol oder 2-Ethylhexanol entstandene Sumpfprodukt handelt.

## Claims

1. A process for esterifying (meth)acrylic acid with an alkanol in the presence of an acidic esterification catalyst, in which unconverted starting compounds and the (meth)acrylate to be formed are separated off by distillation and an oxyester-containing bottom product is formed, wherein the bottom product is separated off and is heated in the presence of an acid to 150-250EC and the pressure is adjusted so that the cleavage products formed under the abovementioned conditions from the oxyesters contained in the bottom product evaporate directly, the process being carried out in the presence of molecular oxygen by passing a stripping gas, which contains molecular oxygen, through the bottom product, as an entraining agent for the cleavage products.

2. A process as claimed in claim 1, wherein a mineral acid, for example sulfuric acid or phosphoric acid, or an alkanesulfonic acid or arylsulfonic acid, for example methanesulfonic acid or p-toluenesulfonic acid, is added to the bottom product.

3. A process as claimed in claim 1 or 2, wherein the amount of acid present is from 1 to 20, preferably from 5 to 15, % by weight, based on the amount of bottom product.

4. A process as claimed in any of claims 1 to 3, wherein reduced pressure (< 1 atm) is used in the cleavage.

5. A process as claimed in any of claims 1 to 4, wherein the cleavage products obtained are recycled directly to the esterification.

6. A process as claimed in any of claims 1 to 5, wherein the bottom product is the bottom product formed in the esterification of n-butanol or 2-ethylhexanol.

## Revendications

1. Procédé d'estérification d'acide (méth)acrylique avec un alcanol en présence d'un catalyseur d'estérification acide, dans lequel les composés de départ n'ayant pas réagis et l'ester (méth)acrylique à former sont séparés par distillation, et il en résulte un produit de fond contenant un oxyester, caractérisé en ce que l'on sépare le produit de fond et on le chauffe à 150-250°C en présence d'acides et en ce que la pression est réglée de sorte que les produits de dissociation obtenus dans les conditions réactionnelles précitées à partir des oxyesters contenus dans le produit de fond s'évaporent immédiatement, le procédé étant mené en présence d'oxygène moléculaire, et où un gaz d'extraction contenant de l'oxygène moléculaire est conduit dans le produit de fond, en tant qu'agent d'entraînement pour les produits de dissociation.

2. Procédé selon la revendication 1, caractérisé en ce que l'on ajoute au produit de fond des acides minéraux, par exemple de l'acide sulfurique ou de l'acide phosphorique, et/ou des acides alkyl- ou arylsulfoniques, par exemple de l'acide méthanesulfonique ou p-toluènesulfonique.

3. Procédé selon la revendication 1 ou 2, caractérisé en ce que les quantités d'acides contenues s'élève à 1-20% en poids, de préférence 5-15% en poids, par rapport à la quantité de produit de fond.

4. Procédé selon l'une quelconque des revendications 1 à 3, caractérisé en ce que l'on utilise une pression réduite (< 1atm) lors de la dissociation.

5. Procédé selon l'une quelconque des revendications 1 à 4, caractérisé en ce que les produits de dissociation obtenus sont immédiatement renvoyés dans l'estérification.

6. Procédé selon l'une quelconque des revendications 1 à 5, caractérisé en ce que le produit de fond est le produit de fond obtenu lors de l'estérification du n-butanol ou du 2-éthylhexanol.
